# EUROPEAN PATENT SPECIFICATION

(11) **EP 1 785 489 B1**
(45) Date of publication and mention of the grant of the patent: **03.03.2010**
(21) Application number: 05758205.8
(22) Date of filing: 06.07.2005
(51) Int. Cl.: C12Q 1/48

(54) **METHOD FOR DETECTION OF PHOSPHORYLATION BY SPR WITH ZINC CHELATE**
VERFAHREN ZUM NACHWEIS DER PHOSPHORYLIERUNG DURCH SPR MIT ZINKCHELAT
PROCEDE POUR LA DETECTION D'UNE PHOSPHORYLATION PAR SPR AVEC UN CHELATE DU ZINC

(30) Priority: 13.07.2004 JP 2004205562; 11.08.2004 JP 2004234635; 08.11.2004 JP 2004323530; 08.11.2004 JP 2004323531
(43) Date of publication of application: 16.05.2007
(73) Proprietor: Toyo Boseki Kabushiki Kaisha, Osaka-shi, Osaka 530-8230 (JP)
(72) Inventor: INAMORI, Kazuki, c/o Toyo Boseki K. K.,, Tsuruga-shi, Fukui 9140047 (JP); NISHIYA, Yoshiaki, c/o Toyo Boseki K. K., Tokyo 1038530 (JP)
(74) Representative: Müller-Boré & Partner Patentanwälte
(86) International application number: PCT/JP2005/012451
(87) International publication number: WO 2006/006456

(56) References cited:
- EP-A- 1 455 189
- EP-A- 1 674 857
- WO-A1-03/053932
- WO-A1-2004/079358
- WO-A1-2005/038442
- JP-A- 2004 305 024
- JP-A- 2004 309 303
- INAMORI KAZUKI ET AL: "Detection and quantification of on-chip phosphorylated peptides by surface plasmon resonance imaging techniques using a phosphate capture molecule." ANALYTICAL CHEMISTRY 1 JUL 2005, vol. 77, no. 13, 1 July 2005 (2005-07-01), pages 3979-3985, XP002469341 ISSN: 0003-2700
- HOUSEMAN BENJAMIN T ET AL: "Peptide chips for the quantitative evaluation of protein kinase activity" NATURE BIOTECHNOLOGY, NATURE PUBLISHING GROUP, NEW YORK, NY, US, vol. 20, no. 3, March 2002 (2002-03), pages 270-274, XP002420888 ISSN: 1087-0156
- TAKEDA H ET AL: "MATRIX-ASSISTED LASER DESORPTION/IONIZATION TIME-OF-FLIGHT MASS SPECTROMETRY OF PHOSPHORYLATED COMPOUNDS USING A NOVEL PHOSPHATE CAPTURE MOLECULE" RAPID COMMUNICATIONS IN MASS SPECTROMETRY, HEYDEN, LONDON, GB, vol. 17, no. 18, 7 August 2003 (2003-08-07), pages 2075-2081, XP002976408 ISSN: 0951-4198
- INAMORI K. ET AL: 'Hyomen Plasmon Kyomei (SSR) Imaging ni yoru Aen Kagobutsu o Mochiita Chip Jo ni Okeru peptide no Phosphation ni kansuru Kenshutsu to Teiryoka' THE MOLECULAR BIOLOGY SOCIETY OF JAPAN NENKAI PROGRAM KOEN YOSHISHU vol. 27, 25 November 2004, page 609, XP002997791
- YAMADA A. ET AL: 'Page 48, Shinki Aen Ion Koteika Affinity Column o Mochiita Phosphation Peptide no Seisei' CHROMATOGRAPHY vol. 24, no. 2, 2003, pages 103 - 104, XP002997792

### SEQUENCE LISTING

<110> Toyo Boseki Kabushiki Kaisya
<120> Method of Detecting Phosphorylation by SPR using zinc chelating Reagent
<130> 050025PC1
<150> PCT/JP2005/012451
   <151> 2004-07-13
<160> 6
<170> PatentIn version 3.1
<210> 1
   <211> 10
   <212> PRT
   <213> Artificial Sequence
<220>
   <223> The sequence of designed peptide described in examples as substrate of PKA, Serine type
<400> 1
   Cys Gly Gly Leu Arg Arg Ala Ser Leu Gly
   1               5                   10
<210> 2
   <211> 10
   <212> PRT
   <213> Artificial Sequence
<220>
   <223> The sequence of designed peptide described in examples as substrate of PKA, Threonine type
<400> 2
   Cys Gly Gly Leu Arg Arg Ala Thr Leu Gly
   1 5 10
<210> 3
   <211> 19
   <212> PRT
   <213> Artificial Sequence
<220>
   <223> The sequence of designed peptide described in examples as substrate of PKC
<400> 3
   Cys Gly Gly Ala Ala Lys Ile Gln Ala Ser Phe Arg Gly His Met Ala Arg Lys Lys
   1 5 10 15
<210> 4
   <211> 10
   <212> PRT
   <213> Artificial Sequence
<220>
   <223> The sequence of designed peptide described in examples as substrate of cSrc kinase
<400> 4
   Cys Gly Ile Tyr Gly Glu Phe Lys Lys Lys
   1 5 10
<210> 5
   <211> 10
   <212> PRT
   <213> Artificial Sequence
<220>
   <223> The sequence of designed peptide described in examples as substrate of cSrc kinase
<400> 5
   Cys Gly Gly Tyr Ile Tyr Gly Ser Phe Lys
   1 5 10
<210> 6
   <211> 10
   <212> PRT
   <213> Artificial sequence
<220>
   <223> The sequence of designed peptide described in examples as negative control for substrate of PKC
<400> 6
   Cys Gly Gly Leu Arg Arg Ala Ala Leu Gly
   1               5                   10

## Description

The present invention relates to a method of analysis of protein kinase activity using an array containing a metal basal plate on which one or more peptides which are recognized by a protein kinase are immobilized. The method detects phosphorylation by phosphorylation on an array and treatment with a chelate compound modified with biotin. In particular, the invention relates to a quick and efficient method which realizes simple analysis of protein kinase activity with analysis technology using surface plasmon resonance (hereinafter, may be referred to as "SPR"). More specifically, the method is a novel analysis technique for protein kinase activity and an application of surface plasmon resonance imaging (hereinafter, may be referred to as "SPR imaging") which produces images based on SPR analysis of interaction between substances.

Recent years have seen dramatic progress in the studies of intercellular signaling. We now have a better understanding about how a signal is transduced from a receptor on a surface of a cell activated by a growth factor, cytokine, etc. to the nucleus. Apart from that, we have increasingly precise knowledge on various signaling pathways which control, for example, cell cycle, adhesion, motion, polarity, morphogenesis, differentiation, and life and death. These signaling pathways do not function individually, but undergo crosstalk, thereby functioning as a system. Cancer and many other disease are attributed to abnormality of the signaling pathways.

It is known that various protein kinases act together in a complicated manner to play important roles in the signaling pathways. It is expected that it will be a great contribution to drug development, clinical application, and other related fields, as well as to basic study in cell biology and pharmacology if the activity of these protein kinases is comprehensively analyzed and their intercellular kinetics are profiled all at once. So far, however, there has been no simple and efficient technique established for simultaneous profiling of kinetics of various protein kinases.

A related technique is reported in, for example, non-patent document 1. The technique uses, for example, a peptide array to evaluate the activity of cSrc kinase, which is a tyrosine kinase. Also, a detection system for phosphorylation reaction using a fluorescence-labeled antibody is reported in, for example, non-patent documents 2 and 3. In the system, arrays are used which contain a glass slide on which substrate peptides for p60 tyrosine kinase, protein kinase A (hereinafter, may be referred to as "PKA"), etc. are immobilized. Further, a detection system for kinase reaction on an array using a radioactive substance ([γ³²P]ATP) is reported in, for example, non-patent documents 4, 5, and 6. None of these prior art documents discloses a sufficient technique for simultaneous and efficient profiling of kinetics of various protein kinases. Moreover, the methods have serious issues. For example, the methods require the use of fluorescent or radioactive substance, which makes the analysis laborious, adds difficulty to handling, and calls for the use of special techniques and equipment.

The detection system using an antibody is applied in various ways. However, no antibody has been discovered which in particular recognizes phosphorylated serine or phosphorylated threonine sufficiently in terms of binding specificity and affinity. The detection system therefore has large problems before we can achieve high precision measurement with it. The antibody is not very advantageous to use as a universal detection system means because different antibodies need to be used to treat different phosphorylated amino acids, and their binding is often highly dependent on the amino acid sequence in the neighborhood of a phosphorylated amino acid.

Houseman et al., Nature Biotechnology, 2002, vol. 20, pages 270 to 274 describes a peptide chip which is usable in activity assays on the non-receptor tyrosine kinase c-Src, and EP 1 674 857 describes a method for measuring a surface plasmon resonance, as well as a noble metal compound usable in said method.
[Non-patent Document 1] Benjamin T. Houseman, et al., Nature Biotechnology, Vol. 20, pp. 270-274 (March 2002)
[Non-patent Document 2] Bioorganic & Medical Chemistry Letters, Vol. 12, pp. 2,085-2,088 (2002)
[Non-patent Document 3] Bioorganic & Medical Chemistry Letters, Vol. 12, pp. 2,079-2,083 (2002)
[Non-patent Document 4] Current Opinion in Biotechnology, Vol. 13, pp. 315-320 (2002)
[Non-patent Document 5] Journal of Biological Chemistry, Vol. 277, pp. 27,839-27,849 (2002)
[Non-patent Document 6] Science, Vol. 289, pp. 1,760-1,763 (2000)

[Figure 1] A drawing showing array patterns of immobilized substrate peptides and results of SPR analysis and SPR imaging.
[Figure 2] A drawing showing results of SPR analysis and SPR imaging.
[Figure 3] A drawing showing array patterns of immobilized substrate peptides and results of SPR analysis and SPR imaging.
[Figure 4] A drawing showing array patterns of immobilized substrate peptides and results of SPR analysis and SPR imaging.
[Figure 5] A drawing showing results of SPR analysis.

The present invention is intended to establish a method of comprehensive profiling of, especially, kinetics of various protein kinases by detecting phosphorylation of a substrate peptide with a quick and easy scheme using an inexpensive substance without the need for a special technique.

The inventors, in view of the circumstances outlined above, have diligently worked and as a result found that for quick and, especially, comprehensive analysis of kinetics of various protein kinases, it is extremely useful to phosphorylate with a protein kinase using an array containing a basal plate carrying thereon a vapor-deposited metal on which is immobilized a peptide which is recognized by the protein kinase and thereafter detect interaction between substances, especially, on the array with SPR imaging upon treating with a biotin-modified chelate compound having a particular molecular weight, and preferably further treating with streptavidin or avidin, which has led to the completion of the invention.

The present invention has the features as characterized in the claims.

The method of the present invention enables very simple and quick analysis of the kinetics of various protein kinases without a need for a special technique. If SPR is used together with the method, there is no need to use a label either, such as a fluorescent or radioactive substance. The use of a chelate compound reduces cost and enables easy handling. Moreover, owing to that use, the method is not affected by the type of phosphorylated amino acid or the amino acid sequence in its neighborhood. These are great advantages over conventional methods. Especially, the present invention enables comprehensive analysis of many types of protein kinase signals, which leads to effective profiling of intercellular protein kinase kinetics upon drug administration or upon the introduction of a gene of which the functions are unknown. Accordingly, the invention is expected to find applications in genome drug development, for example, to analyze functions of new genes and as drug research tools.

The method of detecting a phosphorylated form of a peptide on a basal plate in accordance with the present invention may involve the use of a conventionally well-known label compound, such as a radioactive substance, a fluorescent substance, or a chemiluminescent substance. Preferably, however, an optical detection method is used: e.g., surface plasmon resonance (SPR), ellipsometry, or sum frequency generation (hereinafter, "SFG") spectrometry. Especially preferred among them is SPR because there is no need to obtain a phase difference. SPR is capable of detection of changes in surface film thickness in the order of nanometers by obtaining the intensity of reflected light alone. SPR imaging is preferable because the method allows observation of a large area and also observation of substance interaction using an array.

A peptide can be phosphorylated by applying a nucleoside triphosphate, such as ATP, and a test sample which can contain a protein kinase onto the array of the present invention. Optimal conditions for phosphorylation reaction vary with the type of protein kinase. As an example, the peptide is phosphorylated by adding a nucleoside triphosphate and a test sample which can contain a protein kinase into a buffer and letting them react at about 10 to 40°C, preferably 30 to 40°C, for about 10 minutes to 6 hours, preferably for about 30 to 1 hour. A phosphorylation-facilitating substance, such as cAMP, cGMP, Mg²⁺, or Ca²⁺, phospholipid, may be added, where necessary, to the reaction solution for phosphorylation.

The protein kinases that are targets in kinetics profiling are, for example, the enzymes that phosphorylate a side chain of amino acids, such as tyrosine, serine, threonine, and histidine in proteins. More specific examples are the cGMP-dependent protein kinase family, the cAMP-dependent protein kinase (PKA) family, the myosin light chain kinase family, the protein kinase C (PKC) family, the protein kinase D (PKD) family, the protein kinase B (PKB) family, the protein kinase family belonging to the MAP kinase (MAPK) cascade, the Src tyrosine kinase family, and the receptor tyrosine kinase family.

The peptide of the present invention is aimed at making comprehensive profiling of the kinetics of protein kinases. So, each peptide is preferably phosphorylated by only one protein kinase, and not by other protein kinases. The peptide sequence used as a substrate for the protein kinase may have a publicly known sequence or a suitable one selected from variations of the publicly known sequence. The array of the present invention preferably carries immobilized peptides corresponding to the plurality of protein kinases of which the kinetics need to be understood because the use of only one such array enables the profiling of all the protein kinases. Of course, it is possible to immobilize on each array a peptide corresponding to only one protein kinase and use a required number of arrays when profiling the protein kinases.

Ellipsometry measures the thickness and refractive index of a thin film through changes in polarization caused by interference between reflections of light irradiated onto a sample, one from the front surface of the thin film and the other from the back surface of the thin film. Ellipsometry provides a means of evaluating the ratio of the absolute value of reflectance for primary polarized light and that for secondary polarized light and the ratio of phase shifts for the primary and secondary polarized light. Spectroscopic ellipsometry, or ellipsometry with variable wavelengths, is especially preferred because the method detects changes in the surface film thickness with high sensitivity.

SFG is a secondary non-linear optical effect. The term refers to a phenomenon of two incident beams of light at different frequencies, ω1 and ω2, producing light at ω1+ω2 or ω1-ω2 when mixed in a medium. If visual light and wavelength-variable infrared light are used for ω1 and ω2 respectively, it is possible to carry out vibrational spectroscopy, which resembles infrared spectroscopy. This technique, with its high surface selectivity, enables vibrational spectroscopy on molecules in a single-molecule-thick layer. It is a useful surface analysis method with high sensitivity.

As mentioned above, in one preferred embodiment, the present invention is especially preferably adapted to comprehensively analyze the activity of various protein kinases by SPR. In SPR, a flux of polarized light is irradiated onto metal, producing evanescent waves. The waves then reach to the surface and excite surface plasmons (surface waves). The plasmons consume the light's energy and reduce the intensity of reflection. The resonance angle, or the angle at which the reflection intensity drops by a large amount, changes with the thickness of the layer formed on the metal surface. By observing changes in the resonance angle or changes in the reflection intensity at an angle, one can detect interaction between a target substance (or a collection of substances) immobilized on the metal surface and another substance (or a collection of substances) in a sample. Therefore, SPR requires no labeling with, for example, a fluorescence or radioactive substance. It is a useful assay system capable of real time evaluation.

SPR imaging, an application of SPR, irradiates a flux of polarized light over a large area to analyze images of its reflection. In doing so, the method makes full use of image processing and other related techniques to produce images showing the interaction between the substances. The method enables screening on a chip carrying more than one substance immobilized on it and also high sensitivity observation of the morphology of objects adsorbing to the surface.

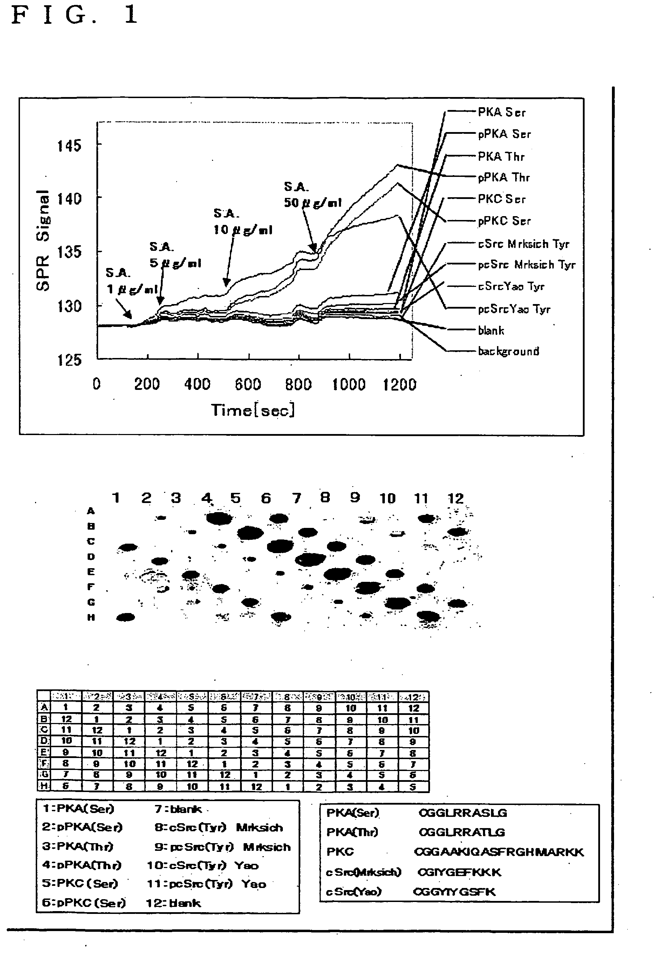

In SPR imaging, a means is needed that irradiates the flux of polarized light with sufficient intensity over a large area of the chip for the analysis of reflection images. Figure 1 shows an example of such a means. The intensity of the polarized light flux is preferably higher because it delivers higher sensor sensitivity.

The light source may be of any type, not limited in any particular manner. A preferable light source, however includes near-infrared wavelengths at which the changes in the SPR resonance angle are especially sensitive. Specific examples include a metal halide lamp, a mercury lamp, a xenon lamp, a halogen lamp, a fluorescent lamp, an incandescent lamp, or another white light emitting source that irradiates over a large area. Especially preferred among them is the halogen lamp, which produces light with the required high intensity and operates from a simple, inexpensive power supply.

The filament of a common white light emitting source has a flaw in that it emits light with non-uniform brightness. If the light emitted from the light source is irradiated as it is, the reflection image is non-uniform in brightness. That would make it difficult to perform screening and evaluate morphology changes. Therefore, the emitted light is preferably guided through a pinhole before being collimated, so that the chip can be illuminated with uniform light. Guiding light through a pinhole is a preferable means of obtaining a flux of light with uniform brightness. The means however undesirably reduces the light's intensity if the light is guided unaided through the pinhole. To address the problem and secure sufficient intensity, a convex lens is preferably disposed between the light source and the pinhole to collect the light before guiding the light through the pinhole.

Since the emitted white light is non-directional, it needs to be collimated through another convex lens before being collected. The light source is disposed about a focal length away from the convex lens to produce parallel light. The first convex lens is disposed with the pinhole being positioned about a focal length away from the lens, so that the light can be collected at the pinhole. The light crosses as it passes inside the pinhole. After that, the light is collimated through a CCTV camera lens. The cross-sectional area of the obtained parallel light is preferably adjusted to 10 to 1,000 mm². This way, it becomes possible to perform screening and morphology observations over a large area.

In imaging the interaction, the flux of polarized light is irradiated to a side of the metal thin film opposite to the side on which the substance or the collection of substances are immobilized. The flux is reflected off the film and guided through an optical interference filter to obtain near-infrared light. The remaining light, in a certain limited region of the spectrum, is captured on a CCD camera.

The optical interference filter preferably has a central wavelength of 600 to 1,000 nm at which SPR shows high sensitivity. The transmittance of the optical interference filter falls to half the maximum value at wavelengths termed half width. The smaller the half width, the sharper the distribution with respect to the wavelength. A smaller half width is therefore preferable. Specifically, the half width is preferably 100 nm or less. The image filtered by the optical interference filter and captured on the CCD camera is fed to a computer on which one can evaluate brightness changes in a part of the image on real time or across the image by image processing. Thus, it becomes possible to perform screening on a chip carrying more than one substance immobilized on it, as well as high sensitivity observation of the morphology of objects adsorbing to the surface.

The SPR chip, or slide, used in the present invention preferably consists of a metal basal plate which has a metal thin film provided on a transparent basal plate. A substance or a collection of substances are chemically or physically immobilized directly or indirectly onto the metal thin film. The basal plate may be made of any material; it is preferably made of a transparent material. Specific examples are glass and plastics, such as polyethylene terephthalate (PET), polycarbonate, and acrylic. Glass is especially preferred among them.

The basal plate is preferably about 0.1 to 20 mm thick, more preferably 1 to 2 mm thick. For the purpose of evaluating the reflection image of the metal thin film, the SPR resonance angle should be as small as possible so that the captured image does not deform. Easy analysis is thus achieved. Therefor, the transparent basal plate, or the transparent basal plate and the prism in contact with that basal plate, preferably has/have a refractive index, nD, of 1.5 or higher.

The metal thin film is made of, for example, gold, silver, copper, aluminum, platinum, or any combination of these metals. Gold is especially preferred. The metal thin film may be fabricated in any manner. Examples of publicly known methods include vapor deposition, sputtering, and ion coating. Vapor deposition is a preferred method. The metal thin film is preferably about 10 to 3,000 Å thick, more preferably about 100 to 600 Å thick.

A preferred concrete example of the present invention is characterized as follows. The examples use an array containing a basal plate carrying thereon a vapor-deposited metal on which is immobilized at least one peptide (preferably two or more peptides) which serves as a substrate for a protein kinase. The array is treated with a solution containing a kinase, such as a homogenized cell solution. The array is then treated with a chelate compound. Their interaction is detected by SPR or SPR imaging among other techniques, In the present invention, the peptide which serves as a substrate for a protein kinase refers to a peptide which is phosphorylated by the protein kinase.

The peptide is not limited in terms of length in any particular manner. The peptide typically contains 100 or less, preferably about 5 to 60, more preferably about 10 to 25 amino acid residues. The peptide may be obtained by chemical synthesis using publicly known techniques or fabricated by genetic engineering techniques. To help the peptide bind to, or part with, a basal plate, the peptide may have biotin, a cysteine residue with a thiol group, or a common tag, such as oligohistidine (His-tag) or glutathione-S-transferase (GST), added to one of the termini.

The substrate peptide may be immobilized on the metal thin film by any method. Preferably, however, a functional group which is readily immobilized on the surface of the metal thin film is introduced prior to the immobilization of the peptide. Examples of such a functional group include an amino group, a mercapto group, a carboxyl group, and an aldehyde group. These functional groups are preferably introduced to the surface of the metal thin film by using a common alkanethiol derivative.

The use of the alkanethiol derivative may be based on the method described by J. M. Brockman, et al., J. Am. Chem. Soc. Vol. 121, pp. 8,044 to 8,051 (1999), so as to immobilize the peptide to the surface with an intervening alkanethiol layer between them and then modify the background with PEG (polyethylene glycol). Also, the peptide may be immobilized after being bound, to the alkanethiol, a derivative in which a functional group listed above is introduced to the end of PEG. This is useful to prevent nonspecific effects and for spacer effect.

Specifically, for example, a water-soluble polymer, like PEG, which has its terminus being modified with carboxymethyl dextran or a carboxyl group is immobilized to the metal thin film to introduce a carboxyl group to the surface. Then, using water-soluble carbodiimide, like EDC (1-ethyl-3,4-dimethylaminopropyl carbodiimide), an amino group in the peptide or protein can react with the activated carboxyl group such as NHS (N-hydroxysuccinimide) ester. Alternatively, the surface is modified with maleimide, and the peptide is immobilized on the surface with intervening amino acid residues containing cysteine or another thiol group. The cysteine residue in that case is preferably added to one of the termini of the peptide. Between these immobilization methods, the latter, which involves an intervening thiol group, is preferred for reduction in nonspecific reactions. These examples are however not limiting the invention in any particular manner.

The aforementioned method of immobilizing a peptide tagged with His-tag or GST is also very simple and useful. In that case, an amino group or a carboxyl group is preferably introduced to the metal surface with intervening alkanethiol as mentioned above, after which NTA (nitrilotriacetic acid) or glutathione is introduced respectively to the surface of the metal thin film. In the case of His-tagging, the substrate is immobilized after treating with nickel chloride an array to which NTA has been introduced.

The present invention uses a chelate compound to monitor the phosphorylation of the substrate on the array specifically and sensitively. A chelate compound is generally a polydentate chelator complex or a chelating reagent complex which are coordinated to zinc, iron, cobalt, palladium, or a like metal ion. Preferable among them are compounds which selectively and reversibly bind to phosphoric acid. A polyamine-zinc complex is more preferable. A binuclear zinc complex containing a polyamine compound as a chelator is even more preferable. A hexamine binuclear zinc(II) complex containing a binuclear zinc(II) complex in its basic structure is yet more preferable.

According to the present invention, the chelate compound is a binuclear zinc complex of formula (I) containing as a chelator a polyamine compound containing 1,3-bis[bis(2-pyridylmethyl)amino]-2-hydroxy propanolate (IUPAC name: 1,3-bis[bis(2-pyridylmethyl)amino]-2-propanolatodizinc(II) complex) as a basic unit (note that the hydroxyl group in the propanol unit is a crosslinking chelator for two divalent zinc ions as an alcoholate.

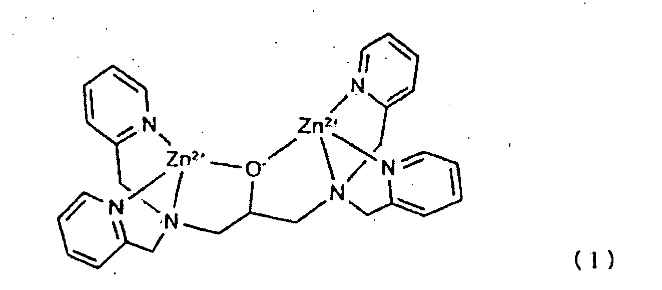

The complex used in the present invention may be synthesized from a commercially available compound by general chemical synthesis technology. As an example, the compound (Zn₂L) of formula (I) can be synthesized from commercially available 1,3-bis[bis(2-pyridylmethyl)amino]-2-hydroxypropane and zinc acetate as follows. A 10-M aqueous solution (0.44 mL) of sodium hydroxide is added to an ethanol solution (100 mL) containing 4.4 mmol of 1,3-bis[bis(2-pyridylmethyl)amino]-2-hydroxypropane. Zinc acetate dihydrate (9.7 mmol) is then added. The solvent is then removed in vacuo to obtain a brown oil. Water (10 mL) is added to this residue to dissolve it. A 1-M aqueous solution of sodium perchlorate (3 equivalent amounts) is added dropwise while being heated to 70°C. The precipitating colorless crystal is filtered out and dried with heat to obtain at high yield the salt of diperchloric acid (Zn₂L-CH₃COO-•2ClO₄-•H₂O) of structural formula (I) to which an acetate ion is bound. The crystal contains one molecule of crystal water.

The ligand and receptor used in the present invention are preferably selected so that they can specifically recognize and bind to each other. Examples of such ligand/receptor combinations are biotin/ avidin, biotin/streptavidin, steroid hormone/steroid hormone receptor, nucleic acid/transcription factor, and single-chain nucleic acid sequence/complementary single-chain nucleic acid sequence. The use of biotin as the ligand and avidin or streptavidin as the receptor is especially preferred among these combinations.

The present invention is characterized by the use of the polyamine-zinc complex described above which is modified with a ligand (e.g., biotin). The ligand is not limited to biotin. It may be steroid hormone, nucleic acid, etc. The ligand is preferably modified with biotin with a straight-chain linker structure intervening between the ligand and the biotin. Its molecular weight is from 500 to 1,000, preferably from 600 to 900, more preferably from 700 to 900. A specific structural example is shown in formula (II). This is however not limiting the invention. It is not preferable in terms of stability and binding efficiency to the phosphoric acid if the biotin-modified polyamine-zinc complex has a molecular weight in excess of 1,000.

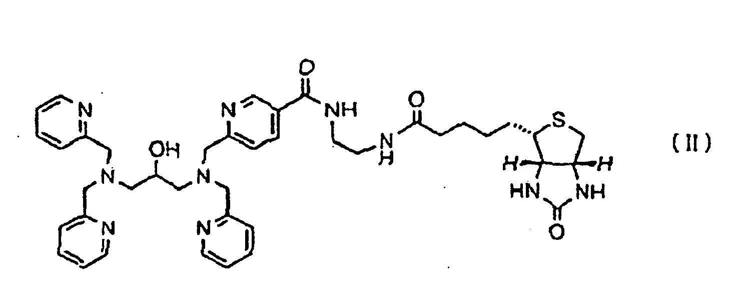

The concentration of the solution of the biotin-modified polyamine-zinc complex used the present invention is not limited in any particular manner and typically ranges from 1 µM to 10 M, preferably from 10 µM to 1 M, more preferably from 10 µM to 10 mM. The array may be treated with the solution in any manner. For example, a sufficient amount of the solution of the biotin-modified polyamine-zinc complex may be dropped so that the solution can spread across the array surface. Alternatively, the array may be immersed in the solution. A further alternative is to use a pump to deliver the solution so that it can contact the array surface. The array may be treated at room temperature or in incubation at 20 to 40°C. The treatment preferably lasts from about 10 minutes to 2 hours, preferably from 30 minutes to 1 hour.

In the present invention; the phosphorylation is generally detected by treating the array only with the biotin-modified polyamine-zinc complex. The detection sensitivity is however expected to increase if the treatment with the biotin-modified complex is followed by a treatment with a receptor, such as avidin or streptavidin. The receptor is not at all limited to avidin or streptavidin and may be a steroid hormone receptor, a transcription factor, a nucleic acid, etc. A treatment with streptavidin is preferred. The concentration of the avidin or streptavidin used in the treatment is not limited in any particular manner and typically ranges from 1 µM to 10 M, preferably from 10 µM to 1 M, more preferably from 10 µM to 10 mM. The array may be treated with the receptor in any manner similarly to the biotin-modified polyamine-zinc complex.

As a more preferred example, the detection sensitivity further increases if the treatment with avidin or streptavidin is accompanied by a treatment with an antibody which recognizes the avidin or streptavidin. The concentration of the antibody used in the treatment is not limited in any particular manner and ranges preferably from 0.01 to 10 µg/mL, more preferably from about 0.1 to µg/mL. The antibody may be either monoclonal or polyclonal. The monoclonal antibody is preferred in view of specificity. The array may be treated with the antibody in any manner similarly to the biotin-modified polyamine-zinc complex, avidin, and streptavidin.

The array may be treated sequentially, first with the biotin-modified polyamine-zinc complex, then followed by either avidin or streptavidin. Alternatively, the array may be treated directly with a complex of the biotin-modified polyamine-zinc complex and either avidin or streptavidin which is prepared in advance. In that case, the array may be further treated with an antibody which recognizes the avidin or streptavidin as in the foregoing case. The complex is preferably prepared by reacting the biotin-modified polyamine-zinc complex and either avidin or streptavidin at a mole ratio of 1:1 to 4:1. The product is preferably refined to remove unreacted substance; the product may however be applied as is.

These chelating compounds are advantageous because they can be synthesized by the method mentioned above at very low cost. They are also stable and easy to use because they can be stored at normal temperatures and also advantageous in distribution. The compounds are far better than especially the detection methods involving an antibody in that the compounds are effective no matter which type of amino acid residue is to be phosphorylated and also that the reaction does not dependent on the amino acid sequence in the neighborhood of the phosphorylated amino acid.

The protein kinase may be one of various tyrosine kinases and serine/threonine kinases. The invention is applicable to any protein kinase.

### [Examples]

The following will describe the present invention more specifically by means of examples. The examples however are not limiting the present invention in any particular manner.

### [Example 1]

### (Immobilization of Peptide)

A four-arm PEG (SUNBRIGHT PTE-100SH made by NOF Corporation), having a thiol group as the terminus functional group, was dissolved in 7 mL of a mixed solution of ethanol and water (ratio = 6:1, concentration = 1 mM). The four-arm PEG molecule weighs 10,000 and has four PEG chains of substantially the same length extending from the center. The molecule exhibits very high hydrophilicity. All the four termini of the PEG are a thiol group. The molecule binds to metals, especially, gold. Chromium was vapor-deposited up to a 3 nm thickness on an SF15 glass slide (18 mm × 18 mm and 2-mm thick). Gold was then vapor-deposited up to a 45 nm thickness. The gold-coated slide was immersed in the solution of the four-arm PEG thiol for 3 hours so that the four-arm PEG thiol could be immobilized on the whole gold basal plate.

The slide was covered with a photo mask and subjected to UV irradiation from a 500 W ultrahigh pressure mercury lamp (made by Ushio, Inc.) for 2 hours to remove the four-arm PEG thiol from UV-irradiated parts of the slide. The photo mask had 8 × 12 = 96 square holes measuring 500 µm on each side. The holes had a center-to-center pitch of 1 mm. The UV light passes through the holes of the photo mask and hits the slide, forming a pattern on the slide. The four-arm PEG remains in those parts which are not irradiated by the light. The parts will serve as a background section, or reference section, of the chip.

The slide was then immersed in a 1-mM ethanol solution of an 8-amino-1-octanethiol hydrochrolide (8-AOT made by Dojindo Laboratories) for 1 hour to form a self-assembled surface of 8-AOT in the UV-irradiated parts. A hetero difunctional polyethylene glycol (NHS-PEG-MAL made by Nektar) was dissolved in a phosphate buffer (20-mM phosphate, 150-mM NaCl, pH = 7.2) to a concentration of 10 mg/mL. The NHS-PEG-MAL molecular weighs 3,400 and has a succinimide (NHS) group and a maleimide (MAL) group at its termini. The mixture was reacted with the 8-AOT on the surface of the gold for 2 hours. The amino groups of the 8-AOT react with the NHS groups of NHS-PEG-MAL. The MAL groups remain unreacted. Thus, the maleimide groups are introduced to the surface with the intervening PEG.

Five substrates for protein kinases (phosphorylated substrates and non-phosphorylated substrates) would be immobilized on the surface thus obtained. Figure 1 shows at its bottom the amino acids sequences of the substrate peptides and a pattern of their immobilization. "Blank" indicates a blank spot where no peptide was immobilized. The substrate peptides were dissolved in respective phosphate buffers (20-mM phosphate, 150-mM NaCl, pH = 7.2) to a rate of 1 mg/mL. The slide was then spotted with 10 nl of each solution using a MultiSPRinter™ spotter (made by Toyobo Co., Ltd.). Next, the slide was left at room temperature for 16 hours in a wet environment to let the immobilization reactions proceed. The maleimide groups formed on the chip surface and the thiol groups of the cysteine residues at the termini of the substrate peptides react, immobilizing the substrate peptides to the surface by covalent bonds.

### (Blocking of Unreacted Maleimide Groups)

The surface on which the substrate peptides had been immobilized was washed in a phosphate buffer. Thereafter, to block unreacted maleimide groups, PEG thiol (SUNBRIGHT MESH-50H made by NOF Corporation) was dissolved in a phosphate buffer (20-mM phosphoric acid, 150-mM NaCl, pH = 7.2) to a concentration of 1 mM, and 300 µL of the solution was dropped on the chip. Reactions were allowed to proceed at room temperature for 30 minutes. The PEG thiol used here had a molecular weight of 5,000.

### (Detection of Phosphate Groups on Array)

After the blocking, the array was washed in PBS and water and treated with a biotin-modified polyamine-zinc complex. The biotin-modified polyamine-zinc complex used was Phos-tag™ BTL-104 (purchased from Nard Institute, Ltd.) of formula (II) below. Phos-tag™ BTL-104 was diluted to 25 µg/mL with a 10-mM HEPES-NaOH buffer (pH = 7.4) containing 0.005% Tween 20, 10% (v/v) ethanol, 0.2 M sodium nitrate, and 1 mM zinc nitrate. The treatment was conducted at room temperature for 1 hour.

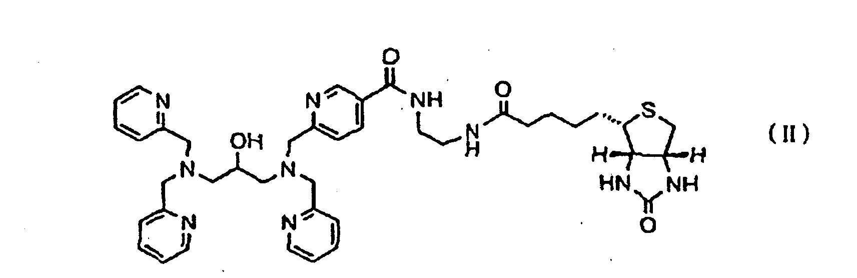

The treated array was washed in PBS and water and placed into an SPR instrument (MultiSPRinter™ made by Toyobo Co., Ltd.) for analysis. The same buffer as above, that is, the 10-mM HEPES-NaOH buffer (pH = 7.4) containing 0.005% Tween 20, 10% (v/v) ethanol, 0.2 M sodium nitrate, and 1 mM zinc nitrate, was used as the running buffer. Streptavidin (made by MolecularProbes) was dissolved in the buffer to prepare 1, 5, 10, 50 µg/mL solutions. The array surface was treated with the solutions sequentially by continuously feeding them from a plunger pump (Model-021 made by Flom Co., Ltd.). The temperature was set to 30°C. The obtained sensorgram is shown at the top of Figure 1. Significant increase in signal intensity is recognized for every phosphorylated substrate when compared to non-phosphorylated substrates although the intensity varies depending on the type of the phosphorylated substrate.

Results of SPR imaging are shown in the center of Figure 1. For the SPR analysis, an image was taken on a CCD camera every 5 seconds. The images taken before and after reaction with streptavidin (hereinafter, may be referred to as "SA") were subjected to subtractive processing using image processing software, Scion Image (Scion Corp.), to produce the illustrated results. Spots are found only at the sites where the phosphorylated substrates were immobilized. That confirms that Phos-tag™ BTL-104 was bound specifically.

### [Example 2]

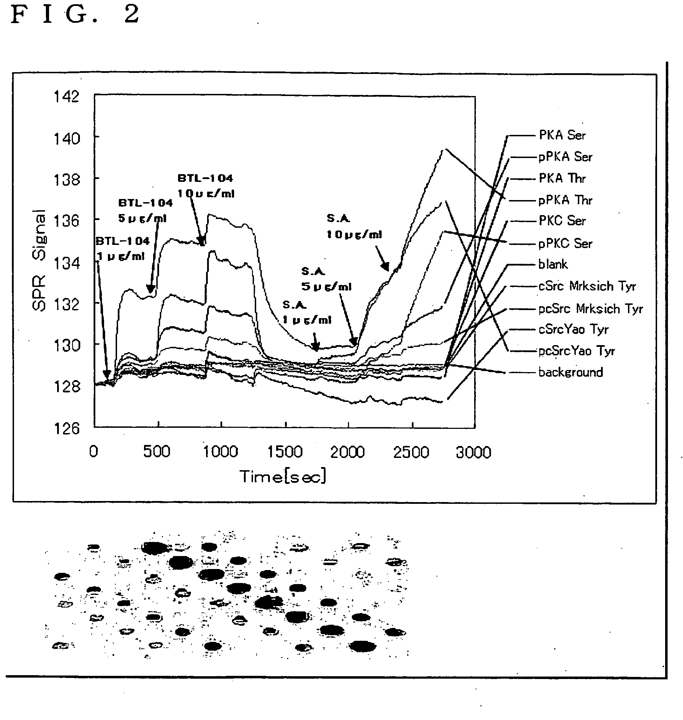

The same operations were carried out as in example 1 up to the blocking of the unreacted maleimide group. The array was then washed in PBS and water and placed into an SPR instrument (MultiSPRinter™ made by Toyobo Co., Ltd.) for analysis. The same running buffer was used as in example 1. Phos-tag™ BTL-104 was dissolved in the running buffer to prepare 1, 5, 10 µg/mL solutions. The array surface was treated with the solutions sequentially by continuously feeding them from a plunger pump (Model-021 made by Flom Co., Ltd.). The temperature was set to 30°C. Thereafter, the array surface was washed in the running buffer being fed from the pump, and further treated with 1, 5, 1.0 µg/mL solutions of streptavidin in this order by feeding them from the pump. The obtained sensorgram is shown at the top of Figure 2. In this case, significant increase in signal intensity is again recognized for all phosphorylated substrates when compared to non-phosphorylated substrates although the intensity varies depending on the type of the phosphorylated substrate. Results of SPR imaging are shown in the center of Figure 2. The SPR imaging was carried out as in example 1. Similar trends are found from the results.

### [Example 3]

### (Immobilization of Peptide)

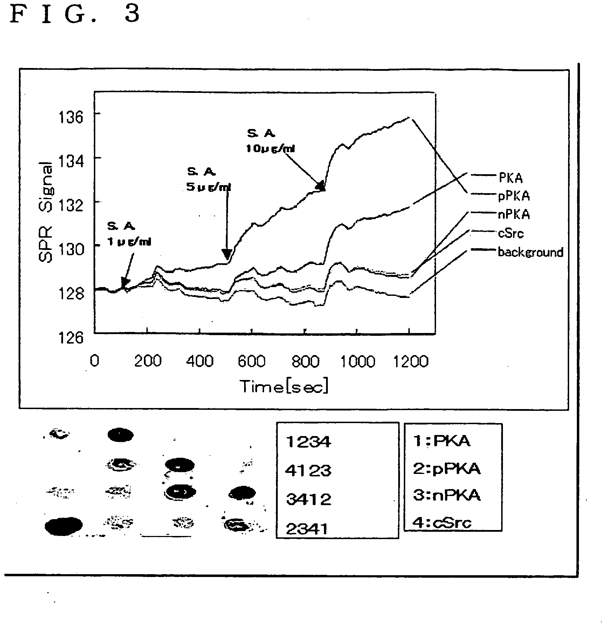

The four-arm PEG thiol was bound to the gold basal plate as in example 1. Following that, the same patterning was carried out as in example 1, except that a different photo mask was used in the UV irradiation. The photo mask had 4 × 4 = 16 square holes measuring 500 µm on each side. The accompanying introduction of amino groups to the array surface and the formation of the maleimide group surface using a crosslinking agent were also carried out as in example 1. The slide was spotted manually with 0.1 µL of each substrate peptide. The substrates used were PKA (protein kinase A) substrate (the same as PKA(Ser) in Figure 1), a positive control (pPKA) of which the serine residue had been phosphorylated, and a negative control (nPKA) of which the serine residue was replaced by an alanine residue. The substrates were immobilized as the pattern which is shown at the bottom of Figure 3. Reactions following the spotting were carried out as in example 1.

### (Blocking of Unreacted Maleimide Groups)

Blocking was carried out exactly as in example 1.

### (Detection of Phosphate Groups on Array)

After the blocking, the array was washed in PBS and water as preparation for phosphorylation with PKA. 400 µL of a PKA solution was dropped on the array to allow reactions to proceed at 30°C for 30 minutes. The PKA solution consisted of 1 µL of a PKA catalytic subunit (made by Promega), 375 µL of a 50-mM Tris-hydrochlorate buffer (pH = 7.4), 20 µL of a 1-M solution of magnesium chloride, and 4 µL of a 10-mM ATP (made by Amersham Bioscience).

The array, having been reacted with PKA, is washed in PBS and water and treated with Phos-tag™ BTL-104 under the same conditions as in example 1. The array was then washed in PBS and water and placed into an SPR instrument (MultiSPRinter™ made by Toyobo Co., Ltd.) for analysis. The same running buffer was used as in example 1. The array was treated with 1, 5, 10 µg/mL solutions of streptavidin in this order by continuous feeding as in example 1. The obtained sensorgram and results of SPR imaging are shown in Figure 3. The strongest binding signal is observed with the positive control. A substantially strong binding signal is observed with the PKA substrate too. Negligible signal changes are observed with the negative control and the blank. These results indicate that the phosphorylation of the PKA substrates on the array was successfully detected.

### [Example 4]

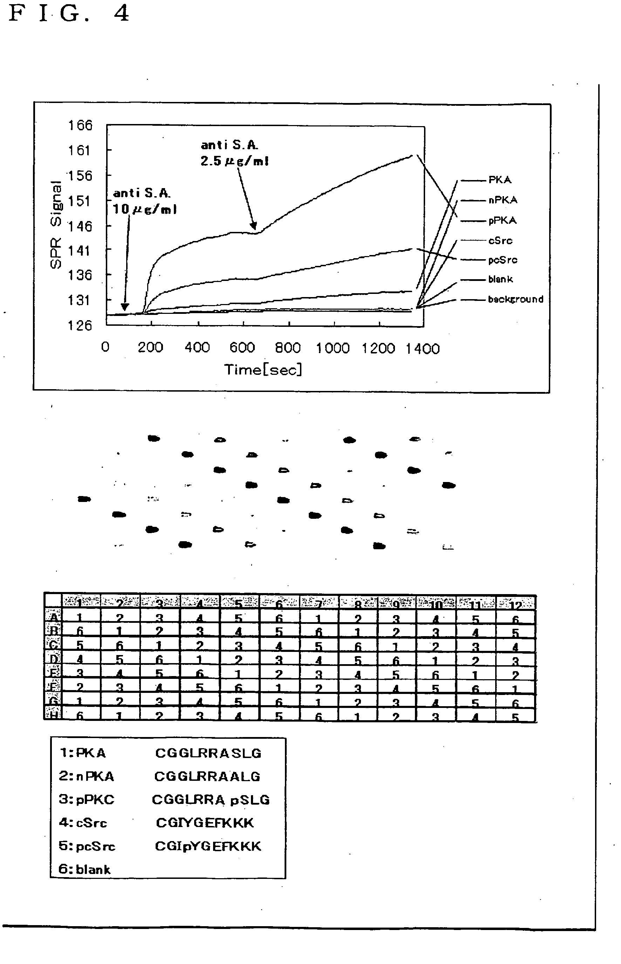

An array was fabricated in 96 spots format by immobilization in the same manner as in example 1, except that the array carried the same PKA substrate and positive and negative controls as those in example 3, as well as a cSrc substrate and its positive control. The substrate pattern is shown at the bottom of Figure 4. Blocking, PKA reaction, and treatment with Phos-tag™ BTL-104 were carried out as in example 3. The array was washed in PBS and water and placed into an SPR instrument (MultiSPRinter™ made by Toyobo Co., Ltd.) for analysis. The same running buffer was used as in example 1. The array was treated with a solution of streptavidin (10 µg/mL) by feeding the solution. After confirming that signals made no more substantial increases, the array was washed in the running buffer being fed, and further treated with a 2.5-µg/mL anti-streptavidin antibody (made by Vector) by feeding. The obtained sensorgram and results of SPR imaging are shown in Figure 4. A strong signal increase is observed with the positive controls for the PKA and cSrc substrates. A substantial signal increase is observed with the PKA substrate too. Almost no signal changes are observed with the negative control and the blank. Signal increases became more distinct as a result of the treatment with the anti-streptavidin antibody. The signal increases also indicate excellent specificity. These results confirm that the anti-streptavidin antibody has an excellent sensitizing effect.

### [Example 5]

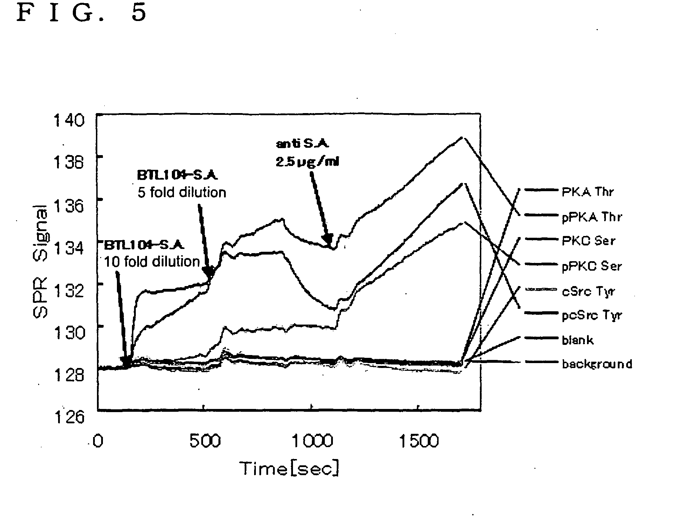

An array on which a PKA substrate (threonine type), a PKC substrate (serine type), and a cSrc substrate (Yao; tyrosine type) shown in Figure 1, both phosphorylated and non-phosphorylated, are immobilized was fabricated as in example 1. Thereafter, the same blocking was carried out as in example 1. The array then was placed into an SPR instrument (MultiSPRinter™ made by Toyobo Co., Ltd.). Meanwhile, a solution Phos-tag™ BTL-104 (50 µg/mL) and a solution of streptavidin (75 µg/mL) were mixed in equal amounts. Reactions were run at room temperature for 30 minutes to produce a complex. This complex solution was diluted 10 fold and 5 fold with the same running buffer as in example 1. The array was treated with the dilute solutions by continuous feeding. After confirming that signals made no more substantial increases, the array was washed in the running buffer being fed, and further treated with a 2.5-µg/mL anti-streptavidin antibody (made by Vector) by feeding. The obtained sensorgram is shown in Figure 5. Specific signal increases are found only with the phosphorylated substrates.

The method of the present invention enables very simple and quick analysis of the kinetics of various protein kinases without the need for a special technique. If SPR is used together with the method, there is no need to use a label either, such as a fluorescent or radioactive substance. The use of a chelate compound reduces cost and enables easy handling. Moreover, owing to that use, the method is not affected by the type of phosphorylated amino acid or the amino acid sequence in its neighborhood. These are great advantages over conventional methods. Especially, the present invention enables comprehensive analysis of many types of protein kinase signals, which leads to effective profiling of intercellular protein kinase kinetics upon drug administration or upon the introduction of a gene of which the functions are unknown. Accordingly, the invention is expected to find applications in genome drug development, for example, to analyze functions of new genes and as drug research tools. The invention hence will make great contributions to industry.

## Claims

1. A method of analysis of protein kinase activity, being **characterized by**, in judging phosphorylation using a peptide which is immobilized on a basal plate, contacting a chelate compound modified with a ligand with the object peptide immobilized on the basal plate, the chelate compound being represented by formula (I):

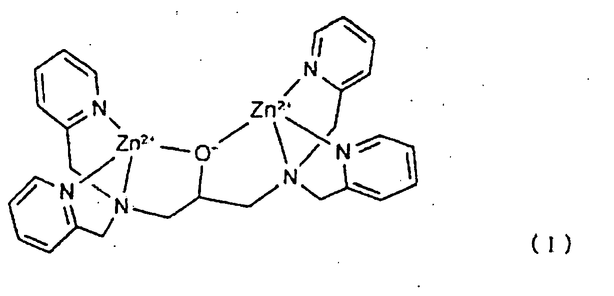

2. The method of claim 1, wherein after the treating with the chelate compound modified with a ligand, the peptide is further treated with a receptor, and the receptor binds specifically to the ligand.

3. The method of either one of claims 1 and 2, wherein after the treating with the receptor, the peptide is further treated with an antibody which recognizes the receptor, and the receptor binds specifically to the ligand.

4. A method of analysis of protein kinase activity, being **characterized by**, in judging phosphorylation using a peptide which is immobilized on a basal plate, forming a complex of a receptor and a chelate compound modified with a ligand and contacting the complex with the object peptide immobilized on the basal plate, the chelate compound being represented by formula (I): 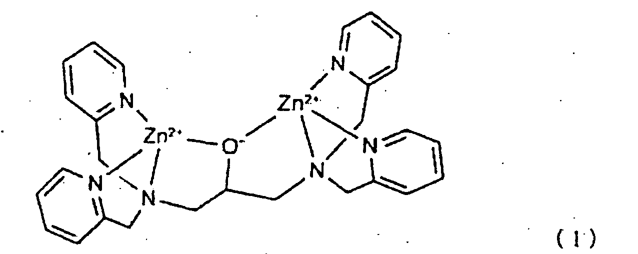 , the receptor binding specifically to the ligand.

5. The method of claim 4, wherein after the treating with the complex, the peptide is further treated with an antibody which recognizes the receptor.

6. The method of any one of claims 1 to 5, wherein the chelate compound modified with a ligand has a molecular weight of 500 to 1,000.

7. The method of claim 6, wherein the chelate compound modified with a ligand has a molecular weight of 600 to 900.

8. The method of any one of claims 1 to 7, wherein: the ligand is biotin; and the receptor specific to that ligand is either avidin or streptavidin.

9. The method of any one of claims 1 to 8, wherein the chelate compound is a polyamine-zinc complex.

10. The method of any one of claims 1 to 9, wherein the chelate compound is a binuclear zinc complex containing a polyamine compound as a chelator.

11. The method of any one of claims 1 to 10, wherein the peptide is a substrate for at least one protein kinase selected from the group consisting of the cGMP-dependent protein kinase family, the cAMP-dependent protein kinase (PKA) family, the myosin light chain kinase family, the protein kinase C (PKC) family, the protein kinase D (PKD) family, the protein kinase B (PKB) family, the protein kinase family belonging to the MAP kinase (MAPK) cascade, the Src tyrosine kinase family, and the receptor tyrosine kinase family.

12. The method of any one of claims 1 to 11, wherein an array is used which contains at least two peptides immobilized on a metal thin film, each peptide being a substrate for a different protein kinase.

13. The method of any one of claims 1 to 12, wherein phosphorylation of at least one peptide which serves as a substrate for at least one protein kinase and is immobilized on a metal thin film in a basal plate of an array is detected by treating the at least one peptide with a nucleoside triphosphate and a test material which can contain a protein kinase.

14. The method of any one of claims 1 to 13, wherein a phosphorylated peptide is detected by surface plasmon resonance (SPR).

15. The method of any one of claims 1 to 14, wherein a phosphorylated peptide is detected by surface plasmon resonance imaging.

16. The method of either one of claims 1 and 4, wherein the basal plate has a metal thin film, and the peptide is immobilized on the metal thin film.

17. The method of any one of claims 1, 4, and 16, wherein the peptide is immobilized on the basal plate, forming an array.

18. The method of claim 17, wherein two or more peptides are immobilized, forming an array.

19. The method of claim 17, wherein two or more peptides are immobilized, forming an array, the two or more peptides each containing an amino acid residue which is a combination of any two or more of serine, threonine, and tyrosine, the residue providing a site where the peptide is phosphorylated.

20. A kit for detecting phosphorylation using a peptide which is immobilized on a basal plate, the kit comprising: a chelate compound modified with a ligand; a receptor specific to the ligand; and an antibody which recognizes the ligand, the chelate compound being represented by formula (I): 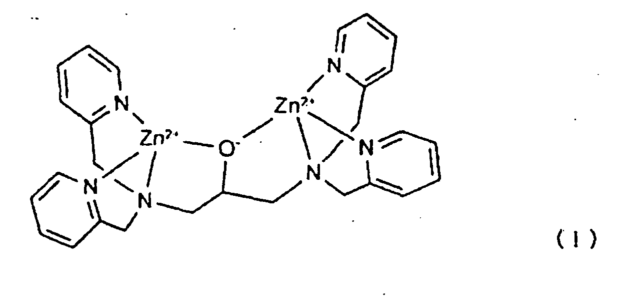 , the ligand/receptor combination being biotin/avidin, biotin/streptavidin, steroid hormone/steroid hormone receptor, nucleic acid/transcription factor, or single-chain nucleic acid sequence/complementary single-chain nucleic acid sequence.

21. The kit of claim 20, wherein: the ligand is biotin; and the receptor specific to that ligand is either avidin or streptavidin.

22. The kit of any one of claims 20 or 21, wherein the chelate compound is a polyamine-zinc complex.

23. A kit for detecting phosphorylation using a peptide which is immobilized on a basal plate, the kit comprising a complex of a receptor and a chelate compound modified with a ligand; and an antibody which recognizes the ligand, the chelate compound being represented by formula (I): 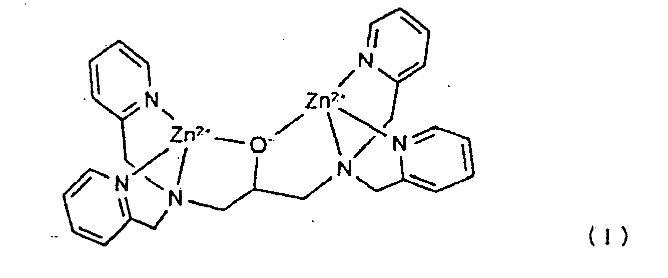 , the ligand/receptor combination being biotin/avidin, biotin/streptavidin, steroid hormone/steroid hormone receptor, nucleic acid/transcription factor, or single-chain nucleic acid sequence/complementary single-chain nucleic acid sequence.

24. The kit of claim 23, wherein: the ligand is biotin; and the receptor specific to that ligand is either avidin or streptavidin.

25. The kit of any one of claims 23 or 24, wherein the chelate compound is a polyamine-zinc complex.

26. The kit of any one of claims 20 to 25, wherein the chelate compound is a binuclear zinc complex containing a polyamine compound as a chelator.

27. The kit of any one of claims 20 to 26, wherein a phosphorylated form of the peptide is detected by surface plasmon resonance (SPR).

28. The kit of any one of claims 20 to 27, wherein a phosphorylated form of the peptide is detected by SPR imaging.

## Patentansprüche

1. Verfahren zur Analyse von Proteinkinase-Aktivität, **gekennzeichnet durch**, zur Beurteilung der Phosphorylierung unter Verwendung eines auf einer Basalplatte immobilisierten Peptids, das Inkontaktbringen einer mit einem Liganden modifizierten Chelatverbindung mit dem Zielpeptid, welches auf die Basalplatte immobilisiert ist, wobei die Chelatverbindung **durch** die Formel (I) dargestellt ist:

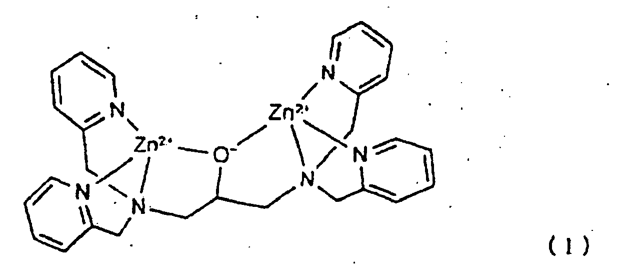

2. Verfahren nach Anspruch 1, wobei nach dem Behandeln mit der mit einem Liganden modifizierten Chelatverbindung, das Peptid weiter mit einem Rezeptor behandelt wird, und der Rezeptor spezifisch an den Liganden bindet.

3. Verfahren nach Anspruch 1 oder 2, wobei nach dem Behandeln mit dem Rezeptor das Peptid weiter mit einem Antikörper behandelt wird, welcher den Rezeptor erkennt, und der Rezeptor spezifisch an den Liganden bindet.

4. Verfahren zur Analyse von Proteinkinase-Aktivität, **gekennzeichnet durch**, zur Beurteilung der Phosphorylierung unter Verwendung eines auf einer Basalplatte immobilisierten Peptids, das Bilden eines Komplexes eines Rezeptors und einer Chelatverbindung, welche mit einem Liganden modifiziert ist, und das Inkontaktbringen des Komplexes mit dem auf der Basalplatte immobilisierten Zielpeptid, wobei die Chelatverbindung **durch** die Formel (I) dargestellt ist: 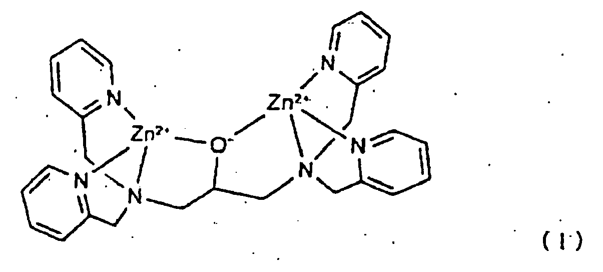 wobei der Rezeptor spezifisch an den Liganden bindet.

5. Verfahren nach Anspruch 4, wobei nach dem Behandeln mit dem Komplex, das Peptid weiter mit einem Antikörper behandelt wird, welcher den Rezeptor erkennt.

6. Verfahren nach einem der Ansprüche 1 bis 5, wobei die mit einem Liganden modifizierte Chelatverbindung ein Molekulargewicht von 500 bis 1000 aufweist.

7. Verfahren nach Anspruch 6, wobei die mit einem Liganden modifizierte Chelatverbindung ein Molekulargewicht von 600 bis 900 aufweist.

8. Verfahren nach einem der Ansprüche 1 bis 7, wobei der Ligand Biotin ist, und der für den Liganden spezifische Rezeptor entweder Avidin oder Streptavidin ist.

9. Verfahren nach einem der Ansprüche 1 bis 8, wobei die Chelatverbindung ein Polyamin-Zink-Komplex ist.

10. Verfahren nach einem der Ansprüche 1 bis 9, wobei die Chelatverbindung ein binuklearer Zink-Komplex ist, welcher eine Polyaminverbindung als einen Chelatbildner enthält.

11. Verfahren nach einem der Ansprüche 1 bis 10, wobei das Peptid ein Substrat für mindestens eine Proteinkinase ist, ausgewählt aus der Gruppe, bestehend aus der cGMP-abhängigen Proteinkinasefamilie, der cAMP-abhängigen Proteinkinasefamilie (PKA), der Myosin-Leichtkettenkinasefamilie, der Proteinkinase C-Familie (PKC), der Proteinkinase D-Familie (PKD), der Proteinkinase B-Familie (PKB), der Proteinkinasefamilie, welche zu der MAP-Kinasekaskade (MAPK) gehört, der Src-Tyrosinkinasefamilie, und der Rezeptortyrosinkinasefamilie.

12. Verfahren nach einem der Ansprüche 1 bis 11, wobei ein Array verwendet wird, welches mindestens zwei Peptide enthält, welche auf einem dünnen Metallfilm immobilisiert sind, wobei jedes Peptid ein Substrat für eine unterschiedliche Proteinkinase ist.

13. Verfahren nach einem der Ansprüche 1 bis 12, wobei die Phosphorylierung von mindestens einem Peptid, welches als ein Substrat für mindestens eine Proteinkinase dient und auf einem dünnen Metallfilm in einer Basalplatte eines Arrays immobilisiert ist, durch das Behandeln des mindestens einen Peptids mit einem Nukleosidtriphosphat und einem Testmaterial, welches eine Proteinkinase enthalten kann, nachgewiesen wird.

14. Verfahren nach einem der Ansprüche 1 bis 13, wobei ein phosphoryliertes Peptid durch Oberflächenplasmonresonanz (SPR) nachgewiesen wird.

15. Verfahren nach einem der Ansprüche 1 bis 14, wobei ein phosphoryliertes Peptid durch Oberflächenplasmonresonanz-Bildgebung nachgewiesen wird.

16. Verfahren nach Anspruch 1 oder 4, wobei die Basalplatte einen dünnen Metallfilm aufweist und das Peptid auf dem dünnen Metallfilm immobilisiert ist.

17. Verfahren nach einem der Ansprüche 1, 4 und 16, wobei das Peptid auf der Basalplatte unter Bildung eines Arrays immobilisiert ist.

18. Verfahren nach Anspruch 17, wobei zwei oder mehr Peptide immobilisiert sind, wobei ein Array gebildet wird.

19. Verfahren nach Anspruch 17, wobei zwei oder mehr Peptide immobilisiert sind, wobei ein Array gebildet wird, wobei die zwei oder mehr Peptide jeweils einen Aminosäurerest enthalten, welcher einer Kombination von beliebigen zwei oder mehr von Serin, Threonin und Tyrosin ist, wobei der Rest eine Stelle bereitstellt, wo das Peptid phosphoryliert wird.

20. Kit zum Nachweisen von Phosphorylierung unter Verwendung eines auf einer Basalplatte immobilisierten Peptids, wobei der Kit umfasst: eine mit einem Liganden modifizierte Chelatverbindung, einen für den Liganden spezifischen Rezeptor, und einen Antikörper, welcher den Liganden erkennt, wobei die Chelatverbindung durch die Formel (I) dargestellt ist: 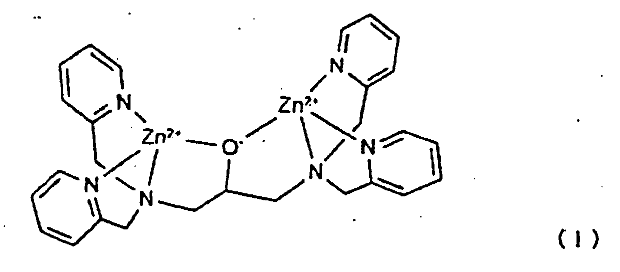 wobei die Ligand/Rezeptor-Kombination Biotin/Avidin, Biotin/Streptavidin, Steroidhormon/Steroidhormonrezeptor, Nukleinsäure/Transkriptionsfaktor oder einzelsträngige Nukleinsäuresequenz/komplementäre einzelsträngige Nukleinsäuresequenz ist.

21. Kit nach Anspruch 20, wobei der Ligand Biotin ist, und der für diese Liganden spezifische Rezeptor entweder Avidin oder Streptavidin ist.

22. Kit nach Anspruch 20 oder 21, wobei die Chelatverbindung ein Polyamin-Zink-Komplex ist.

23. Kit zum Nachweis von Phosphorylierung unter Verwendung eines auf einer Basalplatte immobilisierten Peptids, wobei der Kit einen Komplex eines Rezeptors und einer mit einem Liganden modifizierten Chelatverbindung, und einen Antikörper, welcher den Liganden erkennt, umfasst, wobei die Chelatverbindung durch die Formel (I) dargestellt ist: 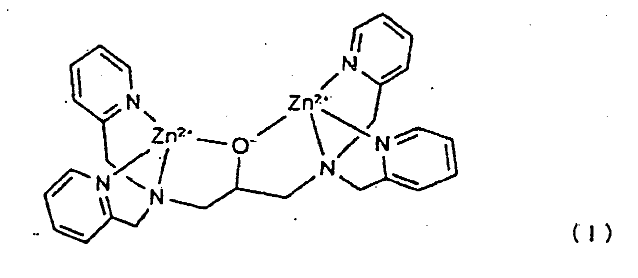 wobei die Ligand/Rezeptor-Kombination Biotin/Avidin, Biotin/Streptavidin, Steroidhormon/Steroidhormonrezeptor, Nukleinsäure/Transkriptionsfaktor, oder einzelsträngige Nukleinsäuresequenz/komplementäre einzelsträngige Nukleinsäuresequenz ist.

24. Kit nach Anspruch 23, wobei der Ligand Biotin ist, und der für diesen Liganden spezifische Rezeptor entweder Avidin oder Streptavidin ist.

25. Kit nach einem der Ansprüche 23 oder 24, wobei die Chelatverbindung ein Polyamin-Zink-Komplex ist.

26. Kit nach einem der Ansprüche 20 bis 25, wobei die Chelatverbindung ein binuklearer Zink-Komplex ist, welcher eine Polyaminverbindung als einen Chelatbildner enthält.

27. Kit nach einem der Ansprüche 20 bis 26, wobei eine phosphorylierte Form des Peptids durch Oberflächenplasmonresonanz (SPR) nachgewiesen wird.

28. Kit nach einem der Ansprüche 20 bis 27, wobei eine phosphorylierte Form des Peptids durch SPR-Bildgebung nachgewiesen wird.

## Revendications

1. Procédé d'analyse de l'activité de la protéine kinase, **caractérisé**, dans l'évaluation de la phosphorylation au moyen d'un peptide qui est immobilisé sur une plaque basale, par la mise en contact d'un composé chélaté modifié avec un ligand avec le peptide objet immobilisé sur la plaque basale, le composé chélaté étant représenté par la formule (I) :

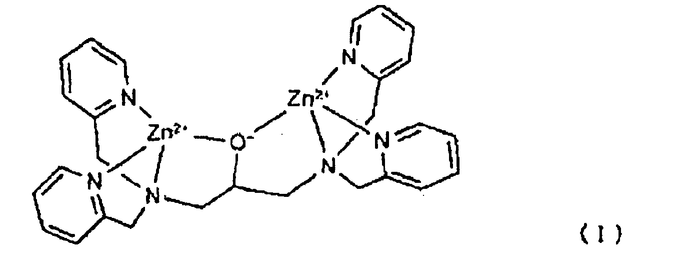

2. Procédé selon la revendication 1, dans lequel après le traitement avec le composé chélaté modifié avec un ligand, le peptide est en outre traité avec un récepteur, et le récepteur se lie spécifiquement au ligand.

3. Procédé selon l'une ou l'autre des revendications 1 et 2, dans lequel après le traitement avec le récepteur, le peptide est en outre traité avec un anticorps qui reconnaît le récepteur, et le récepteur se lie spécifiquement au ligand.

4. Procédé d'analyse de l'activité de la protéine kinase, **caractérisé**, dans l'évaluation de la phosphorylation au moyen d'un peptide qui est immobilisé sur une plaque basale, par la formation d'un complexe d'un récepteur et d'un composé chélaté modifié avec un ligand et la mise en contact du complexe avec le peptide objet immobilisé sur la plaque basale, le composé chélaté étant représenté par la formule (I) : 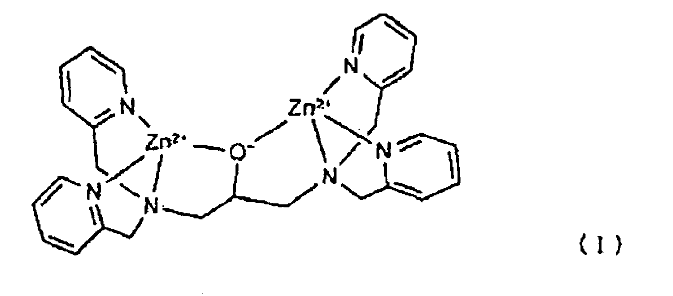 le récepteur se liant spécifiquement au ligand.

5. Procédé selon la revendication 4, dans lequel après le traitement avec le complexe, le peptide est en outre traité avec un anticorps qui reconnaît le récepteur.

6. Procédé selon l'une quelconque des revendications 1 à 5, dans lequel le composé chélaté modifié avec un ligand a une masse moléculaire de 500 à 1000.

7. Procédé selon la revendication 6, dans lequel le composé chélaté modifié avec un ligand a une masse moléculaire de 600 à 900.

8. Procédé selon l'une quelconque des revendications 1 à 7, dans lequel le ligand est une biotine ; et le récepteur spécifique à ce ligand est soit une avidine, soit une streptavidine.

9. Procédé selon l'une quelconque des revendications 1 à 8, dans lequel le composé chélaté est un complexe de polyamine-zinc.

10. Procédé selon l'une quelconque des revendications 1 à 9, dans lequel le composé chélaté est un complexe de zinc binucléaire contenant un composé de polyamine comme chélateur.

11. Procédé selon l'une quelconque des revendications 1 à 10, dans lequel le peptide est un substrat pour au moins une protéine kinase choisie dans le groupe comprenant la famille des protéines kinases dépendantes du cGMP, la famille des protéines kinases dépendantes du cAMP (PKA), la famille des kinases de la chaîne légère de la myosine, la famille des protéines kinases C (PKC), la famille des protéines kinases D (PKD), la famille des protéines kinases B (PKB), la famille des protéines kinases appartenant à la cascade des MAP kinases (MAPK), la famille des tyrosine kinases Src et la famille des récepteurs tyrosine kinases.

12. Procédé selon l'une quelconque des revendications 1 à 11, dans lequel est utilisé un réseau qui contient au moins deux peptides immobilisés sur un film mince métallique, chaque peptide étant un substrat pour une protéine kinase différente.

13. Procédé selon l'une quelconque des revendications 1 à 12, dans lequel la phosphorylation d'au moins un peptide qui sert de substrat pour au moins une protéine kinase et est immobilisé sur un film mince métallique dans une plaque basale d'un réseau est détectée en traitant l'au moins un peptide avec un nucléoside triphosphate et une matière d'essai qui peut contenir une protéine kinase.

14. Procédé selon l'une quelconque des revendications 1 à 13, dans lequel un peptide phosphorylé est détecté par résonance plasmonique de surface (SPR).

15. Procédé selon l'une quelconque des revendications 1 à 14, dans lequel un peptide phosphorylé est détecté par imagerie par résonance plasmonique de surface.

16. Procédé selon l'une ou l'autre des revendications 1 et 4, dans lequel la plaque basale possède un film mince métallique et le peptide est immobilisé sur le film mince métallique.

17. Procédé selon l'une quelconque des revendications 1, 4 et 16, dans lequel le peptide est immobilisé sur la plaque basale, formant un réseau.

18. Procédé selon la revendication 17, dans lequel deux peptides ou plus sont immobilisés, formant un réseau.

19. Procédé selon la revendication 17, dans lequel deux peptides ou plus sont immobilisés, formant un réseau, les deux peptides ou plus contenant chacun un résidu d'acide aminé qui est une combinaison de deux substances quelconques ou plus parmi la sérine, la thréonine et la tyrosine, le résidu fournissant un site où le peptide est phosphorylé.

20. Kit de détection de phosphorylation au moyen d'un peptide qui est immobilisé sur une plaque basale, le kit comprenant : un composé chélaté modifié avec un ligand ; un récepteur spécifique au ligand ; et un anticorps qui reconnaît le ligand, le composé chélaté étant représenté par la formule (I) : 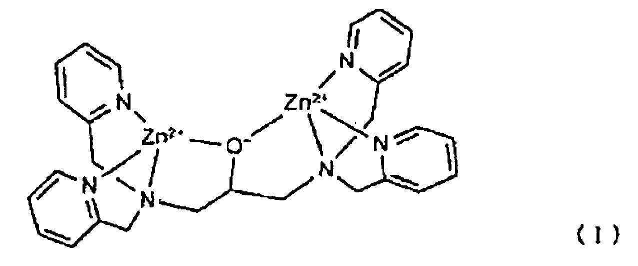 la combinaison ligand/récepteur étant biotine/avidine, biotine/streptavidine, hormone stéroïde/récepteur d'hormone stéroïde, acide nucléique/facteur de transcription ou séquence d'acide nucléique monocaténaire/séquence d'acide nucléique monocaténaire complémentaire.

21. Kit selon la revendication 20, dans lequel le ligand est une biotine ; et le récepteur spécifique à ce ligand est soit une avidine, soit une streptavidine.

22. Kit selon l'une quelconque des revendications 20 ou 21, dans lequel le composé chélaté est un complexe de polyamine-zinc.

23. Kit de détection de phosphorylation au moyen d'un peptide qui est immobilisé sur une plaque basale, le kit comprenant un complexe d'un récepteur et d'un composé chélaté modifié avec un ligand ; et un anticorps qui reconnaît le ligand, le composé chélaté étant représenté par la formule (I) : 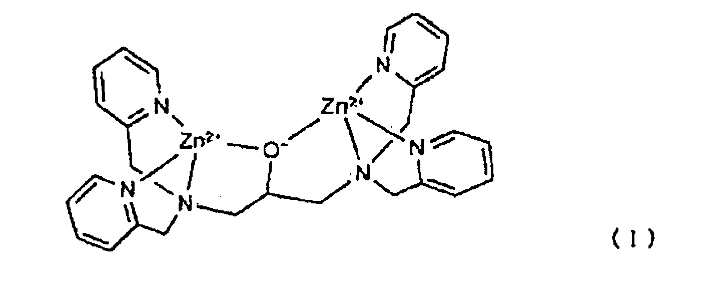 la combinaison ligand/récepteur étant biotine/avidine, biotine/streptavidine, hormone stéroïde/récepteur d'hormone stéroïde, acide nucléique/facteur de transcription ou séquence d'acide nucléique monocaténaire/séquence d'acide nucléique monocaténaire complémentaire.

24. Kit selon la revendication 23, dans lequel : le ligand est une biotine ; et le récepteur spécifique à ce ligand est soit une avidine, soit une streptavidine.

25. Kit selon l'une quelconque des revendications 23 ou 24, dans lequel le composé chélaté est un complexe de polyamine-zinc.

26. Kit selon l'une quelconque des revendications 20 à 25, dans lequel le composé chélaté est un complexe de zinc binucléaire contenant un composé de polyamine comme chélateur.

27. Kit selon l'une quelconque des revendications 20 à 26, dans lequel une forme phosphorylée du peptide est détectée par résonance plasmonique de surface (SPR).

28. Kit selon l'une quelconque des revendications 20 à 27, dans lequel une forme phosphorylée du peptide est détectée par imagerie par SPR.
